# EUROPEAN PATENT SPECIFICATION

(11) **EP 2 769 685 B1**
(45) Date of publication and mention of the grant of the patent: **06.06.2018**
(21) Application number: 14000611.5
(22) Date of filing: 21.02.2014
(51) Int. Cl.: A61B 17/04, A61B 17/06

(54) **Tissue protector suture constructs**
Gewebeschutznahtkonstrukte
Constructions de suture de protection de tissu

(30) Priority: 25.02.2013 US 201361768641 P; 24.04.2013 US 201361815354 P
(43) Date of publication of application: 27.08.2014
(73) Proprietor: Arthrex, Inc., Naples, FL 34108-1945 (US)
(72) Inventor: Schmieding, Reinhold, Naples, FL 34108 (US); Libby, Dustin, Naples, FL 34109 (US); Dreyfuss, Peter J., Naples, FL 34108 (US); Bradley, James P., Pittsburgh, PA 15238 (US)
(74) Representative: Margue, Robert Germain

(56) References cited:
- EP-A1- 1 080 693
- EP-A1- 2 098 253
- EP-A1- 2 201 900
- WO-A1-97/18760
- FR-A1- 2 959 408
- US-A1- 2008 281 422
- US-A1- 2009 306 711

## Description

### FIELD OF THE INVENTION

The present invention relates to the field of surgery and, more particularly, to an improved suture construct for tissue fixation.

### BACKGROUND OF THE INVENTION

Sutures have been known throughout the history of surgery. The variety of materials used to close wounds and attach soft tissue to bone (or soft tissue to soft tissue) has included wires of gold, silver, iron, and steel; dried gut; silk; animal hairs; tree bark and other plant fibers; and, more recently, a wide selection of synthetic compositions such as ultrahigh molecular weight polyethylene (UHMWPE), or the FiberWire® suture disclosed in U.S. Patent No. 6,716,234. EP 2098253 A1 describes a surgical suture comprising a section or core substantially formed of biological material and, optionally, at least a section or portion provided adjacent the biological material. FR 2959408 A1 describes a surgical yarn or suture with a sheath or cover which is movable along the length of the surgical yarn or suture. EP 1080693 A1 describes a surgical suture with puncture closures which may be formed of a polyglycolic acid felt or a Teflon® felt. WO 97/18760 A1 describes a suture body in which a pledget is included or formed, so that when the pledget is fitted into a hole in a body mass, it provides a tight fit. EP 2201900 A1 a suturing construct having a flexible strand with both opposing ends terminating in a same single tail, i.e. the ends are spliced together. US 2008/0281422 describes a collagen patch provided with a series of suture loops around its perimeter, and US 2009/0306711 A1 teaches a fixation member with a channel on the exterior surface to allow a collapsing tube or sleeve to be positioned within the channel.

Improved surgical sutures that would allow the user to use a preferred suture during a surgical procedure with the ability to widen the footprint where the suture will be in contact with tissue are needed to reduce the likelihood of suture tear through.

### SUMMARY OF THE INVENTION

The present invention is defined in and by the appended claims. The present disclosure more generally provides tissue protector constructs and tissue fixation and protection constructs including a flexible strand (for example, suture) for fixation of soft tissue to bone, or of soft tissue to soft tissue, which amplifies the body's healing response created by the introduction of the suture. Fixation of soft tissue to bone (or of soft tissue to soft tissue) is performed using a flexible material (for example, a suture strand, a braid, a suture tape, a stuffed suture, or a combination thereof) with a portion having an expanded footprint to provide a cushion or tissue protector between the flexible strand (suture) and the tissue to be attached. The suture and cushion may be manufactured from materials that have properties to amplify the body's healing response. The cushion may have any shape and geometry that provides cushioning action between the suture and the tissue to be fixated. The cushion may be provided along the length of the flexible strand (i.e., the flexible strand may be provided with a cushion) or the flexible strand may extend from the cushion.

These and other features and advantages of the present disclosure will become apparent from the following description that is provided in connection with the accompanying drawings and illustrated embodiments of the invention.

### BRIEF DESCRIPTION OF THE DRAWINGS

FIGS. 1 and 2 illustrate steps of a method of fixating soft tissue to bone (shoulder repair) with a suture construct formed of suture with a suture cushion (suture with cushion), and in accordance with an exemplary embodiment of the present invention.
FIG. 3 illustrates a perspective view of a suture construct according to a first embodiment of the present invention (with a flexible strand and a suture cushion in the shape of a bar provided with means for holding the cushion to the suture).
FIG. 4 illustrates a front view of the suture construct of FIG. 3.
FIG. 5 illustrates a schematic perspective view of the suture construct of FIG. 3 used for attachment of labrum to the glenoid.
FIG. 6 illustrates a perspective view of a suture construct according to another example of the present disclosure (with a flexible strand and a suture cushion in the shape of a hollow tubular member attached to the suture).
FIG. 7 illustrates a front view of the suture cushion of FIG. 6.
FIG. 8 illustrates a tissue protector suture construct in accordance with another example of the present disclosure.
FIG. 9 illustrates the tissue protector suture construct of FIG. 8 employed for fixation of tissue to bone.
FIG. 10 illustrates a tissue protector of a suture construct according to another example of the present disclosure.
FIG. 11 illustrates a tissue protector suture construct in accordance with yet another example of the present disclosure.
FIG. 12 illustrates the tissue protector suture construct of FIG. 11 employed for fixation of tissue to bone.
FIGS. 13(a)-13(c) illustrate a tissue protector suture construct in accordance with yet another example of the present disclosure.
FIG. 14 illustrates the tissue protector suture construct of FIG. 13(c) employed for fixation of tissue to bone.
FIGS. 15 and 16 illustrate two tissue protector suture constructs in accordance with yet additional examples of the present disclosure.

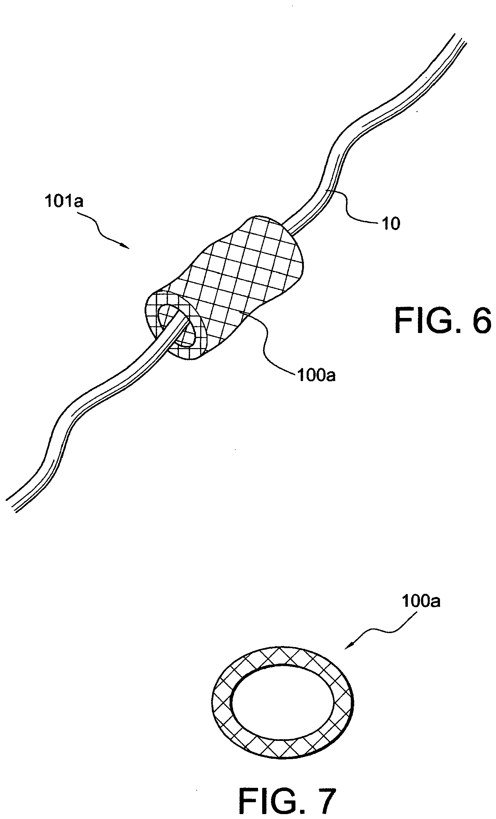

The examples shown in figs. 6 to 16 do not form part of the claimed invention.

### DETAILED DESCRIPTION

The present invention provides constructs for fixation of soft tissue to bone (or of soft tissue to soft tissue) which amplify the healing response created by the introduction of a suture material and the material properties of an attached suture cushion, resulting in the elimination of suture slippage and suture tear associated with the suture.

As detailed below, and according to an exemplary embodiment, fixation of soft tissue to bone is performed using a suture (for example, a suture strand, braid, suture tape, suture with collagen, stuffed suture, or a combination thereof) with a suture cushion arranged along a length of the flexible material, so that the flexible strand (suture) is cushioned against the tissue to be attached. The material properties of the suture cushion amplify the healing response when implanted/secured *in vivo.*

In additional examples, the present disclosure provides constructs for fixation of soft tissue to bone (or of soft tissue to soft tissue) which protect the soft tissue with an attached tissue protector, resulting in the elimination of suture slippage and suture tear associated with passing the suture around the soft tissue. The tissue protector suture construct may be formed of one or more flexible strands (sutures, braids, tapes, or combinations) spliced onto ends of a synthetic or biologic tissue protector (center component). Fixation of tissue to bone is achieved by using the construct passed around tissue and secured into bone with at least one fixation device, for example, at least one suture anchor.

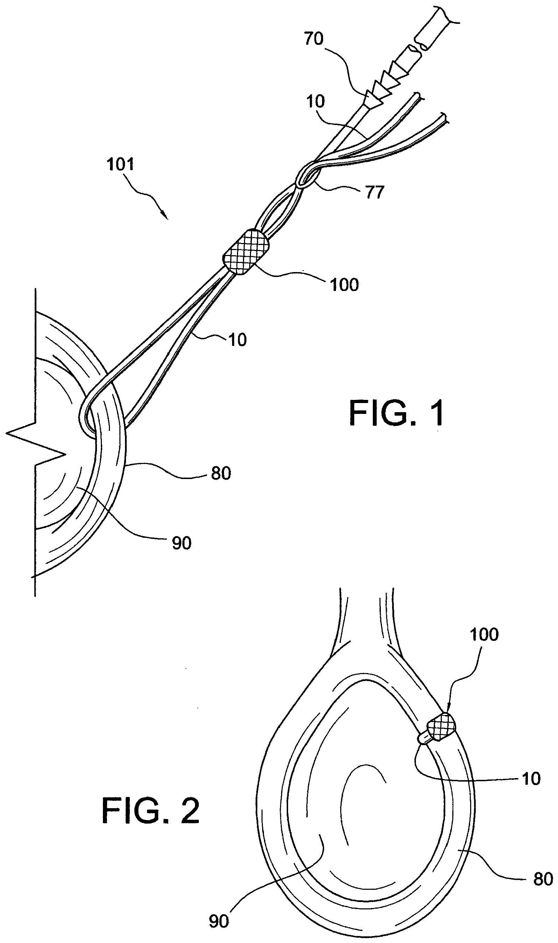

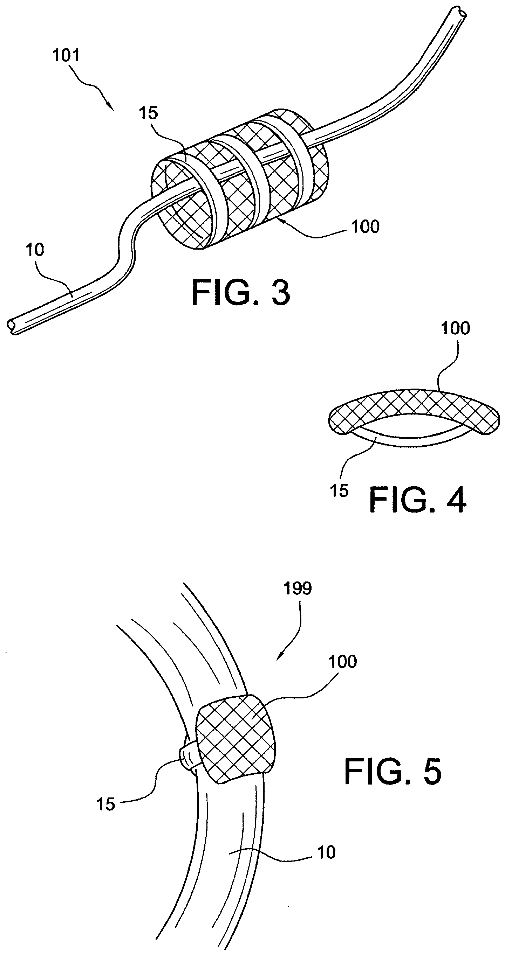

Referring now to the drawings, where like elements are designated by like reference numerals, FIGS. 1-16 illustrates exemplary tissue protector and fixation constructs 101, 101a, 201, 301, 401, 501, 601 of the present disclosure provided with at least one flexible material 10 (flexible strand 10) and a cushion 100, 100a, 200, 200a, 300, 400, 500, 600 arranged along (or extending from) a length of the at least one flexible material. The flexible strand may be removably attached to and/or detached from the cushion or may be provided integral to it. The cushion will be also referred below as to a tissue protector, or tissue protector and fixation construct, or a pad of material.

### FIGS. 1-7

FIGS. 1-7 illustrates exemplary constructs 101, 101a of the present disclosure provided with a flexible material 10 (flexible strand 10) and a suture cushion 100, 100a arranged along a length of the flexible material.

The constructs 101, 101a are formed of a flexible material 10 (for example, a suture strand, a braid, a suture tape, or a combination thereof) with a portion 100, 100a having an expanded footprint to provide a cushion between the flexible strand (suture) and the tissue to be attached. The suture 10 and cushion 100, 100a may be manufactured from materials that have properties to amplify the body's healing response. For example, the cushion 100, 100a may be provided with a medicinal or therapeutic agent, for example, antiseptics, antibiotics, drugs, pharmaceutical agents, hormones and growth materials (for example, autogenous growth factors such as platelet-rich plasma (PRP), autologous factors, autologous-conditioned plasma (ACP)), among many others.

The cushion 100, 100a may have any shape and geometry that provides cushioning action between the suture and the tissue to be fixated. For example, and according to one example of the present disclosure, the cushion 100a is a generally elongated, hollow tubular structure (elongated cylinder or ring) that is securely attached to the flexible strand (suture) and allowed to slide along the length of the flexible strand. In one embodiment, the cushion 100 is a bar having a general rectangular or square configuration and provided with at least one connector to connect the cushion (the suture bar) to the flexible strand (suture) 10.

FIGS. 1 and 2 illustrate a schematic view of a surgical site undergoing a method of fixation of soft tissue to bone (or of soft tissue to soft tissue). In an example only, the surgical site is the shoulder and the tissue is labrum 80 to be attached to glenoid 90 with constructs 101, 101a of the present invention. FIG. 1 also illustrates an exemplary knotless fixation device 70 with an eyelet 77 that allows ends of flexible strand 10 (suture 10) to pass therethrough and aid in the fixation of the labrum 80 to the glenoid 10.

The cushion 100, 100a of the present disclosure may have any shape and geometry to provide increased cushioning action between the suture 10 and the tissue 80 to be fixated. For example, FIGS. 1-5 illustrate various views of a first suture construct 101 with a suture 10 and a suture cushion 100 according to a first embodiment of the present invention. Cushion 100 is a bar having a general rectangular or square configuration and provided with at least one connector 15 to connect the cushion (the bar) to the flexible strand (suture). The at least one connector 15 includes means for holding the cushion 100 to suture 10, for example, a plurality of attachment points that allow suture strands to pass therethrough and hold the cushion (bar) to the flexible strand 10.

FIGS. 6 and 7 illustrate various views of suture construct 101a with flexible strand (suture) 10 and a cushion 100a according to a second example of the present disclosure. According to this example, cushion 100a is a generally elongated, hollow tubular structure (elongated cylinder or ring) that is securely attached to the flexible strand 10 (suture 10) and allowed to slide along the length of the flexible strand 10.

The suture 10 and cushion 100, 100a of constructs 101, 101 a are preferably manufactured from materials that have properties to amplify the body's healing response.

In an exemplary and illustrative embodiment only, the flexible material 10 is a suture strand (for example, high strength suture formed of a ultrahigh molecular weight polyethylene (UHMWPE) braided with polyester, sold under the tradename FiberWire®) or a tape (for example, a collagen tape or a collagen stuffed suture) or any flexible material that allows suturing and/or fixation of tissue to tissue. The flexible material 10 (suture 10) may be provided with optional colored strands to assist surgeons in distinguishing between suture lengths with the trace and suture lengths without the trace. The flexible material 10 may also contain a bioabsorbable material, such as PLLA or one of the other polylactides, for example, and/or may be formed of twisted fibers having strands of a contrasting color added to the braided threads, to make the suture more visible during surgical procedures.

In an exemplary and illustrative embodiment only, the cushion 100, 200 may be formed of suture, suture-like material, foam, polymers, elastic materials, deformable, flexible or rigid materials, or any other material that is body compatible and provides less trauma and irritability to the tissue. The cushion 100, 200 may be optionally provided with a medicinal or therapeutic agent, for example, antiseptics, analgesics, antibiotics, drugs, pharmaceutical agents, hormones, diagnostic agents and growth materials (for example, autogenous growth factors such as platelet-rich plasma (PRP), autologous factors, autologous-conditioned plasma (ACP)), among many others.

The suture construct 101, 101a and cushion 100, 100a of the present invention may be employed in surgical procedures such as rotator cuff repair, Achilles tendon repair, patellar tendon repair, ACL/PCL reconstruction, hip and shoulder reconstruction procedures.

Fixation of soft tissue to bone, such as fixation of labrum 80 to glenoid 90, typically involves the formation of an incision to access the surgical site and then reattachment of the soft tissue. When soft tissue is attached to bone, the surgeon drills a cavity in the bone and inserts a fixation device 70 such as a bone anchor 70. Typically, the bone anchor 70 is formed of metal, composite, plastic or bioabsorbable material, and is held in place by threads or by barbs. If an anchor is employed (such as anchor 70), the anchor typically includes an eyelet 77 through which suture 10 with suture cushion 100, 100a is threaded.

After placing the anchor 70, the surgeon ties the suture 10 through the soft tissue, connecting it to the eyelet 77 of the bone anchor 70, thus re-approximating the soft tissue 80 to the bone 90. The technique is repeated multiple times at different locations in the bone. If multiple sutures are used, however, regrowth of the soft tissue during natural healing is difficult. By providing the cushion 100, 100a attached to the suture, both the footprint of the suture coming into contact with tissue and the healing of tissue 80 are increased, and suture cut through is minimized.

### FIGS. 8-10

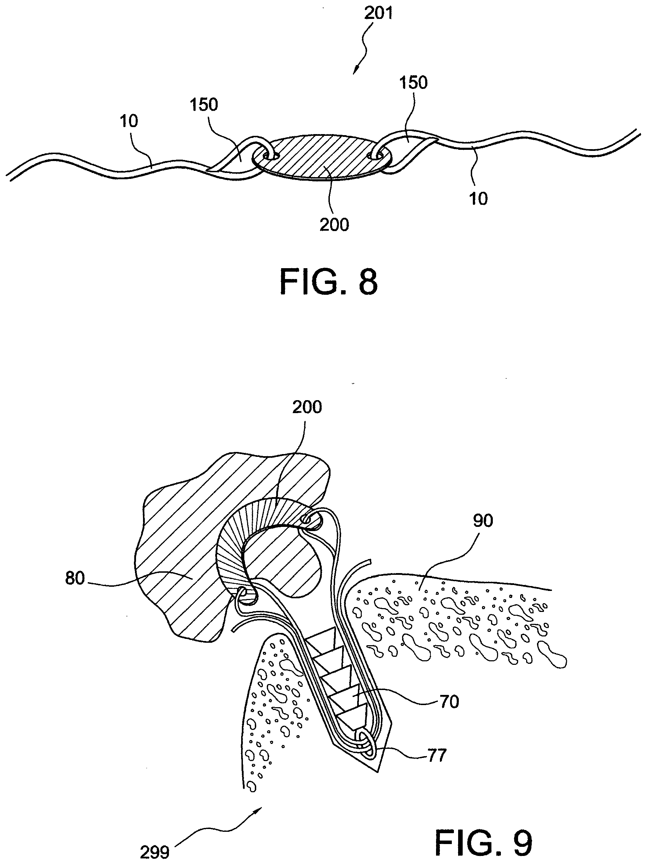

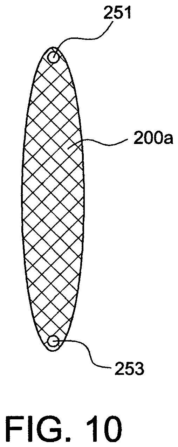

FIGS. 8-10 illustrate exemplary tissue protectors 200, 200a for attachment to flexible materials (flexible strands such as suture) to form a tissue protector suture construct 201. FIGS. 8 and 9 illustrate exemplary suture construct 201 provided with a plurality of flexible materials 10 (flexible strands 10) and a tissue protector 200 attached to the flexible materials. FIG. 10 illustrates another exemplary tissue protector 200a with two small eyelets, each provided at an opposite end of the construct 200a.

Tissue protector construct 201 may be formed of one or more flexible strands 10 (sutures, braids, tapes, or combinations) spliced onto ends of a synthetic or biologic tissue protector (center component) 200, 200a. Fixation of tissue to bone is achieved by using the construct passed around tissue and secured into bone with at least one fixation device, for example, at least one suture anchor.

The tissue protector 200, 200a is preferably wide, soft and strong, and made of various materials and in different shapes and geometries, to provide protection to the tissue to be fixated. The tissue protector 200, 200a may be manufactured from materials that have properties to amplify the body's healing response. For example, the tissue protector 200, 200a may be provided with a medicinal or therapeutic agent, for example, antiseptics, antibiotics, drugs, pharmaceutical agents, hormones and growth materials (for example, autogenous growth factors such as platelet-rich plasma (PRP), autologous factors, autologous-conditioned plasma (ACP)), among many others.

The tissue protector 200, 200a may have any shape and geometry that provides cushioning action to the tissue to be fixated. For example, the tissue protector is a generally elongated structure with an oval or rectangular configuration, and also wide, soft and yet strong, and securely attached to one or more flexible strands (sutures). In yet another example, the tissue protector 200, 200a is provided with at least one connector (for example, an eyesplice or eyesplice interconnection) to connect the tissue protector to the flexible strands (sutures). In yet other examples, the flexible strands (sutures) could just be tied to the tissue protector, without using a connector such as an eyesplice. Alternatively, the flexible strands (sutures) could be attached to the tissue protector by any other means of attachment known in the art.

FIG. 9 illustrates a schematic view of a surgical site undergoing a method of fixation of soft tissue to bone (or of soft tissue to soft tissue). In an example, the surgical site is the shoulder and the tissue is labrum 80 to be attached to glenoid 90 with construct 101. FIG. 9 also illustrates an exemplary knotless fixation device 70 with an eyelet 77 that allows ends of flexible strands 10 (sutures 10) to pass therethrough and aid in the fixation of the labrum 80 to the glenoid 90. The flexible strands may be also secured by employing two fixation devices, for example, two knotless fixation devices 70 with two eyelets 77, *in lieu* of the one fixation device.

FIG. 10 illustrates another exemplary tissue protector 200a of the present disclosure, according to which the tissue protector 200a is provided with two small eyelets 251, 253 at each of the ends of the body of the tissue protector 200a. The one or more flexible strands (sutures) may be simply passed through the ends with eyelets 251, 253 and then fixed with one fixation device (for example, one anchor) or, alternatively, with two fixation devices (for example, two anchors) spaced apart the length of the construct.

The tissue protector 200, 200a may have any shape and geometry that provide increased cushioning action to tissue 80 to be fixated. For example, tissue protector 200, 200a may be any structure that is soft, wide and strong to cushion the tissue to be fixated. Tissue protector 200, 200a may be formed of synthetic or biological material, may be braided or woven, formed of textiles and/or as a mesh or other configurations.

In an example, tissue protector 200 is an elongated, oval structure as shown in FIG. 8 provided with at least one connector 150 for connecting the tissue protector 200 to the flexible strands (sutures). The at least one connector 150 includes means for holding the tissue protector 200 to sutures 10, for example, a plurality of eyesplices 150 or similar structures that allow suture strands 10 to securely attach to and hold the flexible strands 10 to the tissue protector 200. In the example illustrated in FIG. 10, the flexible strands pass directly through the small eyelets 251, 253 provided at opposing ends of the tissue protector 200a and then fixed with one or more fixation devices (for example, one or more suture anchors).

The sutures 10 and tissue protector 200, 200a may be manufactured from materials that have properties to protect the soft tissue and amplify the body's healing response. In an example, at least one of the flexible materials 10 is a suture strand or suture braid with a hollow core (for example, a high strength ultrahigh molecular weight polyethylene (UHMWPE) suture, such as FiberWire® suture) or a tape (for example, a collagen tape or a collagen stuffed suture) or any flexible material that allows splicing, suturing and/or fixation of tissue to tissue. The flexible material 10 (suture 10) may be provided with optional colored strands to assist surgeons in distinguishing between suture lengths with the trace and suture lengths without the trace. The flexible material 10 may also contain a bioabsorbable material, such as PLLA or one of the other polylactides, for example, and/or may be formed of twisted fibers having strands of a contrasting color added to the braided threads, to make the suture more visible during surgical procedures.

In an example, the tissue protector 200, 200a may be formed of suture, suture-like material, foam, polymers, elastic materials, deformable, or flexible materials, synthetic materials, or any other material that is body compatible and provides less trauma and irritability to the tissue. The tissue protector 200, 200a may be optionally provided with a medicinal or therapeutic agent, for example, antiseptics, analgesics, antibiotics, drugs, pharmaceutical agents, hormones, diagnostic agents and growth materials (for example, autogenous growth factors such as platelet-rich plasma (PRP), autologous factors, autologous-conditioned plasma (ACP)), among many others.

The suture construct 201 and tissue protector 200, 200a of the present disclosure may be employed in surgical procedures such as rotator cuff repair, Achilles tendon repair, and patellar tendon repair, among many others.

As shown in FIG. 9, the surgeon places the tissue protector 200 around the soft tissue 80 and ties the sutures 10 through the soft tissue 80, connecting the sutures to the eyelet 77 of the bone anchor 70, thus re-approximating the soft tissue 80 to the bone 90 to achieve repair 299. The technique is repeated multiple times at different locations in the bone. By providing the tissue protector 200, 200a attached to the sutures, both the footprint of the suture coming into contact with tissue and the healing of tissue 80 are increased, and suture cut through is minimized.

In yet additional examples, each flexible strand (suture end) may be connected to a separate fixation device, for example, to two separate suture anchors spaced apart the length of the construct. Thus, the disclosure is not limited to the example shown in FIG. 9 that employs only one fixation device, and has applicability to constructs having the at least one flexible strand/material secured with two or more fixation devices. The tissue protector is cushioned against (protects) the tissue to be fixated/attached. The material properties of the tissue protector amplify the healing response when implanted/secured in vivo.

### FIGS. 11 and 12

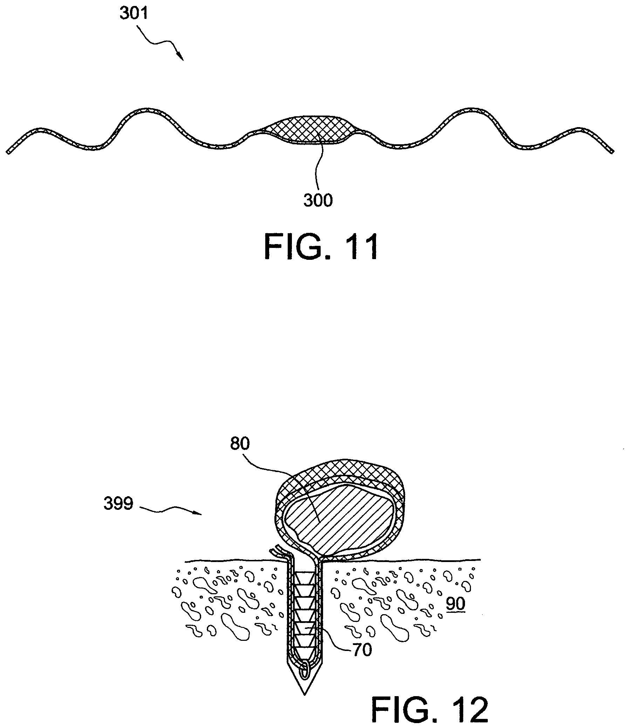

FIGS. 11 and 12 illustrate construct 301 which includes a short, wide flat tape portion 300 with a coreless suture-like tail 10. The flat tape is provided about in the middle area and is flanked by two exemplary suture-like ends. Construct 301 allows a small suture anchor to be used in the repair because the tails are short. During repair 399 (FIG. 12), the broad flat tape is provided over the soft tissue 80 (over the tissue repair) and the suture ends are placed in a small pilot hole formed within bone 90 (and passed through the eyelet of the fixation device/knotless anchor 70).

### FIGS. 13(a)-14

Tissue protector construct 401 is formed of a braided or woven portion 400, 400a provided on one end of a flexible strand 10, for example, a standard round diameter suture 10. The construct has a wider suture footprint and is used to "cinch" or "tag" stitch the soft tissue 80 (for example, labrum 80).

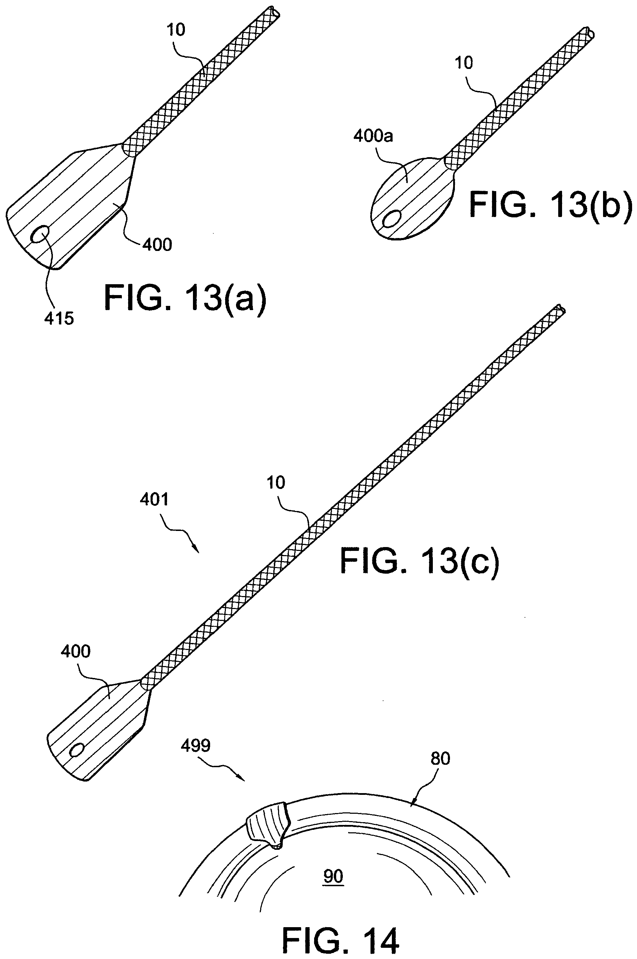

Instead of one strand of suture splicing into itself, suture construct 401 has the braided/woven portion 400 on one end of the round diameter suture. In this "tape-like" portion of suture, there is a reinforced hole 451 (FIG. 13(a)) of which the standard suture will pass through after having passed through the soft tissue.

### FIGS. 15 and 16

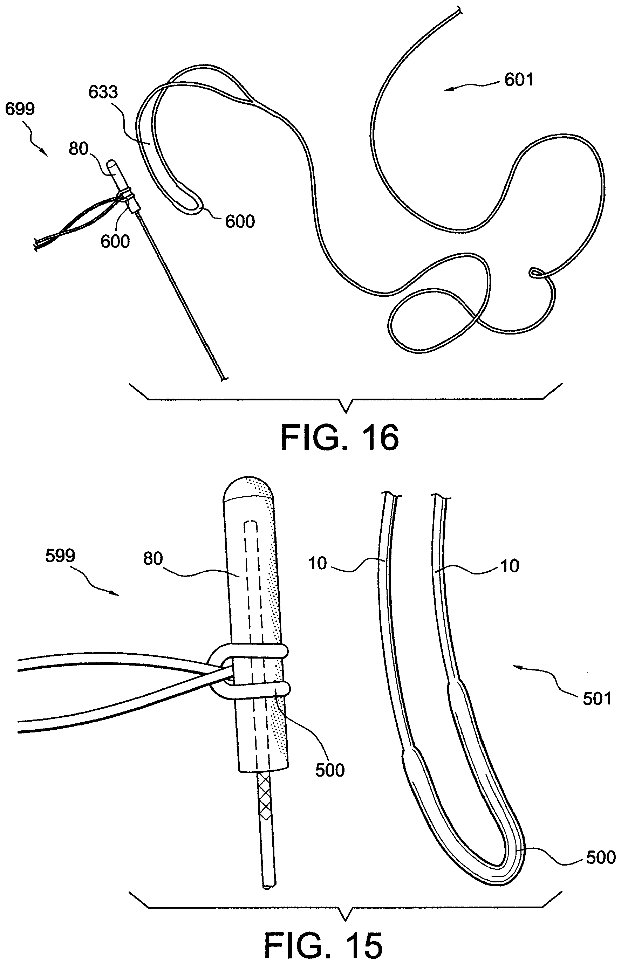

FIGS. 15 and 16 illustrate constructs 501, 601 which are about similar to the construct 301 of FIG. 11 but differ in that the flat tape portion 300 of the construct of FIG. 11 is replaced with a middle portion that is stuffed but not tape. FIG. 15 illustrates, for example, middle portion 500 which has a larger rounder diameter than the ends 10. During repair 599, the stuffed portion 500 is provided over exemplary soft tissue 80 (over the tissue repair) and the suture ends are placed in a hole formed within bone 90, as detailed above with reference to the previously-described constructs.

FIG. 16 illustrates construct 601 which is about similar to the construct 501 of FIG. 15 but differs in that the ends 10 are joined to form flexible loop 633 and the center portion 600 of loop 633 is stuffed. During repair 699, the stuffed portion 600 is provided over exemplary soft tissue 80 (over the tissue repair) and the suture ends are placed in a hole formed within bone 90, as detailed above with reference to the previously-described constructs.

Center portion 500, 600 of constructs 501, 601 is stuffed and has a length of about an inch long (about 2.54 cm long), centered on the end of the loop (for FIG. 16 construct). When a cinch is created around tissue 80, the stuffed (thicker) part 500, 600 is therefore always centered at the repair.

Although the invention has been described with reference to a particular application (i.e., fixation of labrum to glenoid in a shoulder repair), it must be understood that the construct of the present invention has applicability to any type of repairs (any repair in addition to a shoulder repair) and, thus, the invention is not limited by this exemplary-only embodiment.

Although the invention has been described with reference to only one cushion (body member, or pad, or tissue protector) provided along a length of flexible material, the present invention also contemplates embodiments wherein a plurality of cushions are provided along one or more flexible strands (sutures).

Fixation device 70 detailed above may be a knotless fixation device, for example, a knotless suture anchor such as the two-piece Arthrex PushLock® anchor, disclosed in U.S. Patent No. 7,329,272, or an Arthrex SwiveLock® anchor, disclosed in U.S. Patent No. 8,012,174. As detailed in these patents and applications, a knotless suture anchor is formed of an implant with a distal eyelet (which may be closed or forked, and which engages and secures at least one flexible strand) and a fixation device (a cannulated screw) that is advanced over the implant body to secure the implant and the flexible strand into a bone hole or socket, and to approximate soft tissue to bone. The implant may be rotatably attached to (and/or may swivel relative to) a tip of a driver assembly that is pre-loaded with the separate, cannulated fixation device (cannulated screw).

While the present invention is described herein with reference to illustrative embodiments for particular applications, it should be understood that the invention is not limited thereto. Those having ordinary skill in the art and access to the teachings provided herein will recognize additional modifications, applications, embodiments and substitution of equivalents. Accordingly, the invention is not to be considered as limited by the foregoing description.

## Claims

1. A tissue protector construct, comprising:
at least one flexible strand (10); and
a cushion (100) attached to the at least one flexible strand (101) and provided along the length of the at least one flexible strand, wherein the cushion (100) is slidable along the at least one flexible strand (10) to provide cushioning action between the at least one flexible strand (10) and soft tissue to be fixated or attached,
and the cushion (100) is attached to the at least one flexible strand (10) by at least one connector (15, 150); **characterised in that** the cushion (100) is in the form of a rectangle or a square.

2. A tissue protector construct according to claim 1, wherein the cushion (100) is removably attached to the at least one flexible strand (10).

3. A tissue protector construct according to any of claims 1 to 2, wherein the cushion (100) is formed of resorbable material.

4. A tissue protector construct according to any of claims 1 to 3, wherein the strand (10) is a suture, a braid, a fiber strand, a suture tape, or a collagen tape.

5. A tissue protector construct according to any of claims 1 to 4, wherein the cushion (100) is formed of a material selected from the group consisting of suture polymers, elastic materials, foam and suture-like materials.

6. A tissue protector construct according to any of claims 1 to 5, wherein the cushion (100) further comprises a biological component selected from the group consisting of platelet-rich plasma and autologous conditioned plasma.

7. A tissue protector construct according to any of claims 1 to 6, wherein the cushion (100) is a pad of material.

8. A tissue protection and fixation construct consisting of a tissue protector construct according to any of claims 1 to 7, and a knotless fixation device (70) provided with a closed eyelet (77) at its most distal end to allow the flexible strand (10) to pass thereto and to anchor the flexible strand (10) into bone.

9. A tissue protection and fixation construct according to claim 8, wherein the knotless fixation device (70) is a swivel anchor.

## Patentansprüche

1. Gewebeschutzkonstrukt, umfassend:
mindestens einen flexiblen Strang (10); und
ein Kissen (100), das am flexiblen Strang (101) angebracht ist und entlang dem flexiblen Strang vorgesehen ist, wobei das Kissen (100) entlang dem flexiblen Strang (10) verschoben werden kann, um zwischen dem flexiblen Strang (10) und dem zu fixierenden oder zu befestigenden weichen Gewebe eine polsternde Wirkung zu entfalten, und das Kissen (100) am flexiblen Strang (10) durch mindestens einen Verbinder (15, 150) befestigt ist;
**dadurch gekennzeichnet, dass** das Kissen (100) eine rechteckige oder quadratische Form aufweist.

2. Gewebeschutzkonstrukt gemäß Anspruch 1, wobei das Kissen (100) am flexiblen Strang (10) lösbar befestigt ist.

3. Gewebeschutzkonstrukt gemäß einem der Ansprüche 1 bis 2, wobei das Kissen (100) aus resorbierbarem Material gebildet ist.

4. Gewebeschutzkonstrukt gemäß einem der Ansprüche 1 bis 3, wobei es sich bei dem Strang (10) um einen Faden, ein Geflecht, einen Faserstrang, ein Fadenband oder ein Kollagenband handelt.

5. Gewebeschutzkonstrukt gemäß einem der Ansprüche 1 bis 4, wobei das Kissen (100) aus einem der folgenden Materialien gebildet ist: Polymerfäden, elastische Materialien, Schaumstoffe und fadenähnliche Materialien.

6. Gewebeschutzkonstrukt gemäß einem der Ansprüche 1 bis 5, wobei das Kissen (100) ferner eine aus Plasma bestehende biologische Komponente umfasst, die reich an Blutplättchen oder autolog konditioniert ist.

7. Gewebeschutzkonstrukt gemäß einem der Ansprüche 1 bis 6, wobei das Kissen (100) aus einem Polstermaterial gebildet ist.

8. Gewebeschutz- und Fixierungskonstrukt, bestehend aus einem Gewebeschutzkonstrukt gemäß einem der Ansprüche 1 bis 7 und einer knotenlosen Fixierungsvorrichtung (70), die an ihrem distalen Ende eine geschlossene Öse (77) aufweist, damit der flexible Strang (10) dorthin gelangen und der flexible Strang (10) im Knochen verankert werden kann.

9. Gewebeschutz- und Fixierungskonstrukt gemäß Anspruch 8, wobei es sich bei der knotenlosen Fixationsvorrichtung (70) um einen Drehanker handelt.

## Revendications

1. Une construction de protecteur de tissu, comprenant :
au moins un toron flexible (10) ; et
un coussin (100) fixé à au moins un toron flexible (101) et fourni le long d'au moins un toron flexible, dans lequel le coussin (100) est coulissable le long d'au moins un toron flexible (10) pour fournir une action d'amortissement entre au moins un toron flexible (10) et un tissu mou à fixer ou à attacher, et le coussin (100) est fixé à au moins un toron flexible (10) par au moins un connecteur (15, 150) ;
**caractérisé en ce que** le coussin (100) a la forme d'un rectangle ou d'un carré.

2. Une construction de protecteur de tissu selon la revendication 1, dans laquelle le coussin (100) est fixé de manière amovible à au moins un toron flexible (10).

3. Une construction de protecteur de tissu selon l'une quelconque des revendications 1 à 2, dans laquelle le coussin (100) est formé d'un matériau résorbable.

4. Une construction de protecteur de tissu selon l'une quelconque des revendications 1 à 3, dans laquelle le toron (10) est une suture, une tresse, un toron de fibres, un ruban de suture ou un ruban de collagène.

5. Une construction de protecteur de tissu selon l'une quelconque des revendications 1 à 4, dans laquelle le coussin (100) est formé d'un matériau choisi dans le groupe constitué de polymères de suture, de matériaux élastiques, de mousse et de matériaux de suture.

6. Une construction de protecteur de tissu selon l'une quelconque des revendications 1 à 5, dans laquelle le coussin (100) comprend en outre un composant biologique choisi dans le groupe constitué d'un plasma riche en plaquettes et d'un plasma conditionné autologue.

7. Une construction de protecteur de tissu selon l'une quelconque des revendications 1 à 6, dans laquelle le coussin (100) est un tampon de matériau.

8. Une construction de protection et de fixation de tissu consistant en une construction de protection de tissu selon l'une quelconque des revendications 1 à 7, et un dispositif de fixation sans noeud (70) muni d'un oeillet fermé (77) à son extrémité la plus distale pour permettre au toron flexible (10) d'y passer et d'ancrer le toron flexible (10) dans l'os.

9. Une construction de protection et de fixation de tissu selon la revendication 8, dans laquelle le dispositif de fixation sans noeud (70) est une ancre pivotante.
